# EUROPEAN PATENT APPLICATION

(11) **EP 4 821 914 A1**
(43) Date of publication of application: **14.10.2026**
(21) Application number: 24901177.6
(22) Date of filing: 11.10.2024
(51) Int. Cl.: C07C 227/16, C07C 227/34, C07C 229/22, C07C 229/76

(54) **NOVEL METHOD FOR PRODUCING L-SERINE**

(30) Priority: 08.12.2023 KR 20230177306
(71) Applicant: Astrogen, Inc., Daegu 41072 (KR)
(72) Inventor: HWANG, Su-Kyeong, Daegu 41072 (KR); RYU, Hyung Chul, Daegu 41072 (KR)
(74) Representative: Dehns
(86) International application number: PCT/KR2024/096338
(87) International publication number: WO 2025/121991

(57) **Abstract**

The present invention relates to a novel method for synthesizing L-serine from glycine without using toxic substances or bacterial strains such as Escherichia coli.

## Description

### Technical Field

The present invention relates to novel manufacturing method of L-serine.

### Background Art

Considering that serine is necessary for biosynthesizing purines and pyrimidines, serine is an important amino acid for metabolizing a material. Serine is a precursor of various amino acids such as glycine and cysteine as well as bacterial tryptophan. Serine is also a precursor of many other metabolites, such as folic acids, sphingolipids, which are major donors of a fragment that is consisting of a single carbon atom in a biosynthesis.

Among them, in particular, L-serine is involved in many metabolic systems and plays an important role in the normal growth and development of nerve cells. L-serine plays an essential role in cell proliferation by synthesizing bases which serve as materials for deoxyribonucleic acid (DNA). L-serine also acts as a precursor in the synthesis of glutathione, and thus, L-serine plays an important role in antioxidation. Phospholipids such as phosphatidylserine and sphingosine, which are derived from L-serine, are the main components of the myelin sheath that surrounds nerve cells and allow neural information to be transmitted at a high speed. In addition, L-serine activates N-methyl-D-aspartate (NMDA) receptor among neurotransmitters.

Meanwhile, traditionally, L-serine has been produced either through fermentation or by first producing a DL-serine mixture using mercury, and then obtaining pure L-serine through optical separation.

Advances in Biochemical Engineering/Biotechnology 79 (2003) 1 discloses a method for producing L-serine using *Hyphomicrobium* sp. NCIB10099, a Gram-negative bacterium, to produce 45 g/L of L-serine from 100 g/L of glycine with 88 g/L of methanol over 3 days. However, this method is not commercially utilized due to issues with the bacterial strain and reduced commercial efficiency, making it unsuitable for pharmaceutical or food applications.

Another known method is disclosed in U.S. Patent No. 5382517 which describes a method for producing L-serine with an 89% yield by fermenting glycine and formaldehyde solution (485 g/L) with *E. coli* MT-10350 at 50°C for 35 hours. However, the use of *E. coli* in this manner is not suitable for health supplements or pharmaceuticals. Additionally, methods using fermentation require an additional analysis regarding harmful bacteria during drug approval, and thus require management difficulties such as the need for separate clean areas. In addition, formaldehyde has severe carcinogenicity and extremely low allowable residue levels, and thus, formaldehyde cannot be used for pharmaceutical manufacturing in Korea.

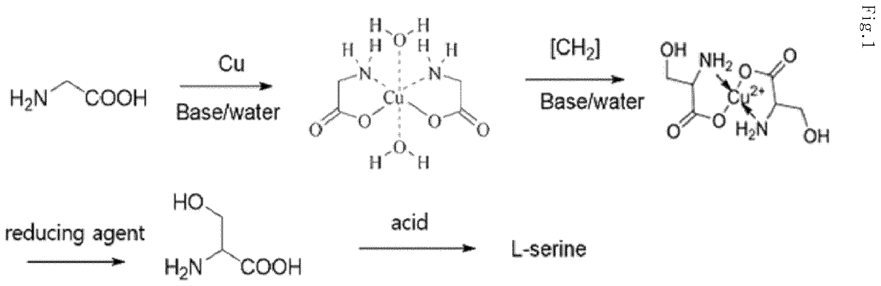

Furthermore, *Org. Synth*. 1940, 20, 81 discloses a method for producing DL-serine as shown in Figure 1. According to this method, an overall yield of about 30 to 40% can be achieved based on mercuric acetate. However, concerns arise from the potential residue of mercury which is a Class I management heavy metal that is highly toxic to the human body. In addition, the use of corrosive substances like HBr necessitates expensive equipment without a risk of corrosion, and there are challenges related to leakage risks and increased wastewater costs, making large-scale production difficult. Moreover, the strong odor of acrylate which is used as a starting material, severely limits its industrial use.

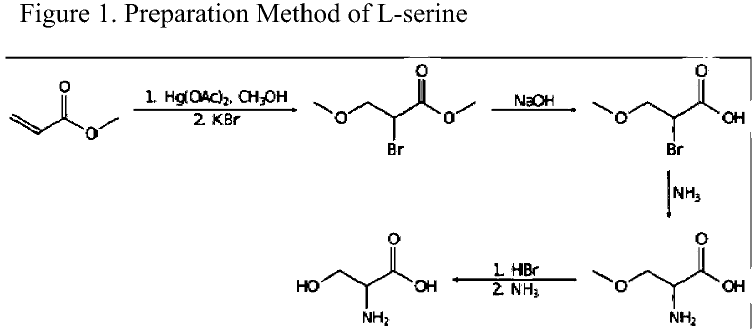

Another method for producing serine, disclosed in Chinese Patent Publication No. 105037060, involves reacting ammonium carbonate and ethylene glycol in an aqueous solution at a high pressure of 3 MPa. However, the reaction under high-pressure requires specialized high-pressure equipment and poses a significant risk of explosion, making it unsuitable for industrial manufacturing method.

In addition, Japanese Patent Publication No. 1993-140056 describes a method for obtaining racemic serine using a costly platinum catalyst to react highly flammable oxygen with aminodiol. This method also pertains difficulties in terms of economic feasibility and stability for industrial production.

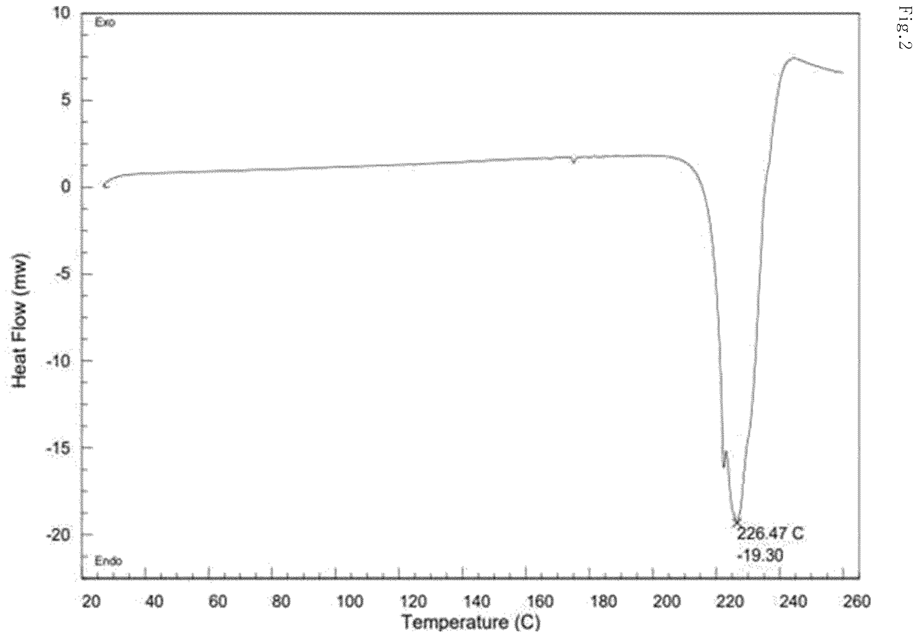

The Bulletin of the Chemical Society of Japan (2002), 75(6), 1383-1384 describes a method for producing racemic serine from glycine using a Cu(II) complex as a catalyst. However, the Cu complex used in this method, as shown in Figure 2, has a very complex synthesis process, making it unsuitable for commercial use in the industrial production of serine.

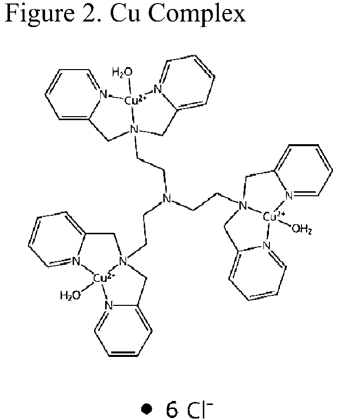

### Documents of Related Art

(Patent Document 1) United States Registered Patent No. 5382517
(Patent Document 2) Chinese Published Patent No. 105037060
(Patent Document 3) Japanese Published Patent No. 1993-140056
(Non-Patent Document 1) Advances in Biochemical Engineering/Biotechnology 79 (2003) 1
(Non-Patent Document 2) Org. Synth. 1940, 20, 81
(Non-Patent Document 3) Bulletin of the Chemical Society of Japan (2002), 75(6), 1383-1384

### Disclosure of Invention

### Technical Problem

The inventors of the subject invention have tried to provide a novel method which synthesizes L-serine from glycine without using toxic substances or microorganisms such as *E. coli.*

### Solution to Problem

To achieve the above objective, the present inventio provides a method for producing L-serine, which includes a) a step of subjecting glycine and a copper compound to chelation to form a glycine-copper complex; b) a step of subjecting a carbon source to a reaction with the complex of the a) step to form a complex into which hydroxymethyl is introduced; c) a step of treating the complex of the b) step with a reducing agent to form DL-serine; and d) a step of optically separating the DL-serine of the c) step to collect L-serine.

### Advantageous Effects of Invention

The method of producing L-serine according to the present invention is a method that makes it possible to synthesize L-serine from glycine without using a toxic substance or bacteria such as *E. coli*, where the method has a characteristic of industrial applicability.

### Brief Description of figures

Fig. 1 illustrates one embodiment of a production method for L-serine according to the present invention.
Fig. 2 shows the results obtained by analyzing L-serine obtained in accordance to the production method of the present invention with differential scanning calorimetry (DSC).

### Best Mode for Carrying out the Invention

Unless otherwise defined, all technical and scientific terms used in this specification have the same meaning as understood by a person skilled in the art to which the present invention belongs to. Generally, the nomenclature used in this specification is well-known and commonly used in the field.

One aspect of the present invention relates to a production method for L-serine.

Embodiment of the present invention relates to a production method for L-serine, which includes a) a step of subjecting glycine and a copper compound to chelation to form a glycine-copper complex; b) a step of subjecting a carbon source to a reaction with the complex of the a) step to form a complex into which hydroxymethyl is introduced; c) a step of treating the complex of the b) step with a reducing agent to form DL-serine; and d) a step of optically separating the DL-serine of the c) step to collect L-serine. One embodiment of the production method according to the present invention is illustrated in Fig. 1.

In the present invention, "L-serine" is L-serine used in the present invention is a chemical compound denoted by Formula 1 below.

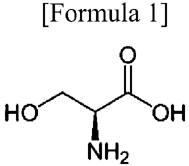

In the present specification, the term "purified" means that in a case where a separation target is separated, the separation target has a purity of 90% or more, preferably a purity of 95% or more, more preferably a purity of 99% or more, and most preferably a purity of 99.9% or more.

In the present invention, the a) step is a step of subjecting glycine and a copper compound to chelation to form a glycine-copper complex.

The a) step may be conducted by adding glycine, a copper, a base and purified water.

Copper that forms a complex with glycine in the a) step is, as a copper complex, generally used in a variety of industrial manufacturing methods and is easily purchased, and any one selected from the group consisting of CuSO₄, CuOAc₂, CuCl₂, CuI, CuBr, CuBr₂, and a hydrate thereof, or one or more thereof may be used as a mixture.

The glycine that is used in the a) step can be used such that the amount thereof is 5 kg to 700 kg, 10 kg to 600 kg, 20 kg to 500 kg, 30 kg to 400 kg, 40 kg to 300 kg, 50 kg to 200 kg, 60 kg to 150 kg, or 70 kg to 100 kg, depending on the size of the manufacturing device. The amount of the copper compound may be 0.3 to 1 equivalents, preferably 0.4 to 0.7 equivalents, and most preferably 0.45 to 0.55 equivalents with respect to the amount of the glycine to be used.

As the base that is used in the a) step, any one or more selected from the group consisting of ammonia water, ammonia gas, sodium hydroxide, potassium hydroxide, calcium hydroxide, triethylamine, diethylamine, sodium carbonate, potassium carbonate, sodium bicarbonate, potassium bicarbonate, and a mixture thereof may be used. The amount of the base may be 0.6 to 4 equivalents, preferably 0.8 to 3 equivalents, and most preferably 1.0 to 2.5 equivalents with respect to the amount of glycine.

In this case, the amount of purified water may be 6 to 30 times, preferably 4 to 20 times, and most preferably 3 to 10 times the weight of glycine; however, the amount of purified water is not limited thereto. In a case where too much amount of purified water is used, it is less likely to be easily handled in the process, and the amount of waste water to be discarded after the process increases. Therefore, it is important to use a minimum amount of purified water to ensure economic feasibility.

In addition, it is difficult to form a glycine-copper complex in a case of increasing temperature, which decreases the yield. Therefore, room temperature or a temperature of 40°C is preferred.

The glycine-copper complex of the a) step may be such as one that has the structure of Formula 2 below.

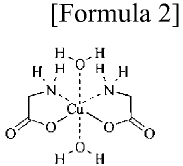

In the present invention, the b) step is a step of subjecting a carbon source to a reaction with the complex of the a) step to form a complex into which hydroxymethyl is introduced.

The b) step may be such as one that is carried out by adding a carbon source, a base, and purified water.

Specifically, the b) step is a process of introducing one carbon into the complex of the a) step and then introducing a hydroxy group to convert the complex into a copper complex of racemic serine having the structure of Formula 3 below.

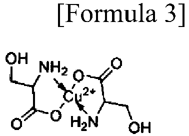

As the base that is used in the b) step, any one or more selected from the group consisting of ammonia water, ammonia gas, sodium hydroxide, potassium hydroxide, calcium hydroxide, triethylamine, diethylamine, sodium carbonate, potassium carbonate, sodium bicarbonate, potassium bicarbonate, and a mixture thereof may be used.

The amount of the purified water that is used in the b) step may be 6 to 25 times, preferably 5 to 20 times, and most preferably 4 to 15 times the weight of the glycine-copper complex having the structure of Formula 2; however, the amount of the purified water is not limited thereto.

The carbon source that is used in the b) step may be any one or more selected from the group consisting of a formaldehyde dilution solution, paraformaldehyde, 1,3-dioxolane, 1.3-dioxane, and hexamidine, and it may be preferably paraformaldehyde or hexamidine. For the formaldehyde, a 15% to 35% aqueous solution thereof may be used as a diluted solution; however, the formaldehyde is not limited thereto. The carbon source may be 1 to 5.0 equivalents, preferably 1.5 to 4.0 equivalents, and most preferably 2.0 to 3.5 equivalents with respect to the content of the glycine-copper complex having the structure of Formula 2; however, it is not limited thereto.

Regarding the reaction temperature, the reaction is carried out at room temperature; however, the temperature may be raised to room temperature to 40°C or lower to carry out stirring for a smooth reaction, and the reaction time may be 1 to 18 hours, preferably 2 to 14 hours, and most preferably 3 to 12 hours.

In the present invention, the c) step is a step of treating the complex of the step b) with a reducing agent to form DL-serine.

The reducing agent that is used in the c) step may be sodium borohydride (NaBH₄), lithium aluminum hydride (LiAlH₄), or sodium sulfide (Na₂S), and it may be most preferably sodium sulfide. The sodium sulfide may be 1 to 5 equivalents, preferably 1.2 to 4.0 equivalents, and most preferably 1.5 to 3.5 equivalents with respect to the content of the copper complex of racemic serine having the structure of Formula 3; however, it is not limited thereto.

The solvent that is used in the c) step may be one or more selected from the group consisting of purified water, methanol, ethanol, isopropanol, butanol, and hexanol, may be preferably purified water, methanol, ethanol, or isopropanol, and most preferably purified water or methanol. In addition, for smooth stirring in a case of using the organic solvent, water that accounts for 10% to 40% of the total amount of solvent to be used may be mixed and used. The total amount of solvent to be used may be 6 to 30 times, preferably 5 to 20 times, and most preferably 4 to 12 times the weight of the copper complex of racemic serine having the structure of Formula 3; however, it is not limited thereto.

Regarding the reaction temperature, the reaction is carried out at room temperature; however, the temperature may be raised to room temperature to 50°C or lower to carry out stirring for a smooth reaction. The reaction time may be 3 to 24 hours, preferably 4 to 18 hours, and most preferably 6 to 12 hours.

A step of carrying out cooling by carrying out reflux with purified water may be further included in order to completely remove the copper once removed from the complex after the c) step is carried out. In this case, the amount of purified water to be used may be 2 to 10 times and preferably 4 to 7 times the amount of DL-serine obtained in the c) step.

In the present invention, the d) step is a step of optically separating the DL-serine in the step c) to collect L-serine.

In the d) step, in order to selectively obtain only L-serine, an L-serine-acid salt of Formula 4 may be obtained by using several acids that are optical division promoting substances.

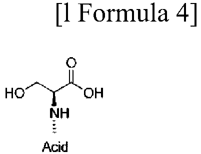

The acid that is used in the d) step may be any one or more selected from the group consisting of tartaric acid, malic acid, mandelic acid, and camphorsulfonic acid, or may be one or more selected from the group consisting of (+)-tartaric acid, (-)-malic acid, (-)-mandelic acid, and (+)-camphor-10-sulfonic acid. The acid may be 0.85 to 1.5 equivalents, preferably 0.90 to 1.25 equivalents, and most preferably 0.95 to 1.15 equivalents of racemic DL-serine obtained in the c) step, and the reaction temperature may be 40°C to 100°C, preferably 50°C to 90°C, and most preferably 55°C to 85°C.

The reaction solvent in the d) step may be any one or more selected from the group consisting of ethyl acetate, toluene, methyl-t-butyl ether, acetic acid, acetone, tetrahydrofuran, isopropanol, methanol, and ethanol, and preferably ethyl acetate, acetic acid, or isopropanol. The amount of solvent to be used is 6 to 20 times, preferably 5 to 15 times, and most preferably 4 to 12 times the weight of DL-serine; however, it is not limited thereto. The reaction time may be 1 to 12 hours, preferably 2 to 10 hours, and most preferably 3 to 6 hours.

The process of filtering the generated L-serine acid salt and washing away the remaining acid can be carried out through any of the filtration methods used in industrial manufacturing methods, such as decompression filtration, centrifugation, and filter drying. It is possible to use an organic solvent such that the amount thereof is 1 to 8 times, preferably 2 to 7 times, and most preferably 3 to 6 times the used DL-serine. In this case, it is preferable that the organic solvent has a moisture content of 1% or less in order to prevent the yield decrease. The organic solvent may be any one or more selected from the group consisting of acetone, methanol, ethanol, isopropanol, butanol, hexanol, ethyl acetate, toluene, methyl-t-butyl ether, methylene chloride, cyclohexane, and pentane, and may be preferably acetone, methanol, ethanol, or isopropanol.

In order to obtain only pure L-serine, a process of neutralizing L-serine acid salt of Formula 4 using a base may be additionally included, and in this case, a base capable of being used may be any one or more selected from the group consisting of ammonia water, ammonia gas, sodium hydroxide, potassium hydroxide, calcium hydroxide, triethylamine, diethylamine, sodium carbonate, potassium carbonate, sodium bicarbonate, potassium bicarbonate, and a mixture thereof. In this case, the pH may be between 6 and 7.

The drying temperature may be between 20°C and 80°C, preferably between 30°C and 70°C, and most preferably between 40°C and 60°C, and a vacuum or hot air drying method may be used; however, they are not limited thereto.

Hereinafter, the present invention will be described in more detail according to the following examples. However, the following examples are only for the exemplary description of the present invention, and thus the scope of the present invention is not limited thereto. The examples of the present invention are provided to more fully explain the present invention to a person of ordinary skill in the field to which the present invention pertains.

### Example 1. Production of L-glycine-copper complex

### Example 1.1. Production of L-glycine-copper complex using anhydrous CuSO₄

75.0 kg of glycine (purchased from EOS, China) and 1,000 L of purified water were put into the reactor at room temperature and stirred for 10 minutes. Thereafter, 156 kg of anhydrous CuSO₄ (purchased from Pure & Applied, China) and 80 kg of NaOH (purchased from Heungchang Chemical) were added thereto, followed by stirring for 6 hours at room temperature. After the completion of the reaction, the temperature was lowered to about 10°C, the formed solid was subjected to decompression filtration and then washed three times with 100 kg of purified water. Vacuum drying was carried out at 50°C for 10 hr to obtain a target compound as a blue solid (yield: 83.5%).

### Example 1.2. Production of L-glycine-copper complex using 5-hydrated CuSO₄ (CuSO₄·H₂O)

75.0 kg of glycine (purchased from EOS, China) and 1,200 L of purified water were put into the reactor at room temperature and stirred for 10 minutes. Thereafter, 249 kg of CuSO₄·H₂O (purchased from Pure & Applied, China) and 80 kg of NaOH (purchased from Heungchang Chemical) were added thereto, followed by stirring for 6 hours at room temperature. After the completion of the reaction, the temperature was lowered to about 10°C, the formed solid was subjected to decompression filtration and then washed three times with 100 kg of purified water. Vacuum drying was carried out at 50°C for 10 hr to obtain a target compound as a blue solid (yield: 82.0%).

### Example 2. Production of DL-serine copper complex

### Example 2-1. Production of DL-serine copper complex

60 kg of the glycine-copper complex obtained in Example 1, 15 kg of paraformaldehyde (purchased from EOS, China), and 500 kg of purified water were put into a reactor at room temperature and stirred for 10 minutes. Thereafter, 45 kg of NaOH was slowly added thereto while maintaining room temperature. After carrying out stirring for 1 hour at room temperature, stirring was carried out for 12 hours in a state where the temperature was raised to about 40°C, and then cooling was carried out to approximately room temperature after filtration. The formed solid was subjected to decompression filtration and then washed three times with 100 kg of purified water. Vacuum drying was carried out at 50°C for 10 hr to obtain a target compound as a blue solid (yield: 77.0%).

### Example 2-2. Production of DL-serine-copper complex

A target compound as a blue solid (yield: 74.0%) was obtained by the same method as in Example 2-1, except that 12.5 kg of hexamidine (imported from Reliable chem, China) was used instead of paraformaldehyde in Example 2-1.

### Example 3. Production of DL-serine

30 kg of the substance obtained in Example 2 and 240 kg of purified water were put into a reactor at room temperature and stirred for 10 minutes. Then, 8.8 kg of sodium sulfide (Na₂S) (purchased from EOS, China) was slowly added thereto while maintaining room temperature. After carrying out stirring for 2 hours at room temperature, stirring was carried out at a temperature of about 45°C for 8 hours, and then cooling was carried out to about 5°C. After adding the formed solid to 150 kg of purified water, the temperature was raised to carry out reflux for 2 hours, and then the temperature was gradually lowered to about 5°C. After decompression filtration, the filtrate was washed once with 10 kg of purified water and twice with 20 kg of ethanol. Vacuum drying was carried out at 50°C for 10 hr to obtain a target compound as a white solid (yield: 79%).

### Example 4. Production of L-Serine

### Example 4-1. Production of L-serine using camphorsulfonic acid

10 kg of the DL-serine obtained in Example 3 and 23.2 kg of L-(-)-camphorsulfonic acid (purchased from EOS, China) were put into a reactor, and 100 kg of acetic acid (purchased from Samchun Pure Chemical Co., Ltd.) was added thereto and dissolved. It was heated to about 80°C to 90°C and stirred for 3 hours. Thereafter, the mixture was cooled to about 5°C to 10°C, stirred for 1 hour, and then filtered to obtain a L-serine-camphorsulfonic acid salt as a white solid. The obtained solid was dissolved in 120 L of anhydrous ethanol, and 28% to 30% ammonia water was added thereto to adjust the pH to about 6.0 to 7.0. The reactant was stirred at room temperature for 1.5 hours, the temperature was lowered to about 5°C to 10°C, additional stirring was carried out for 3 hours, and then filtration was carried out to obtain a crude L-serine product as a white solid. Thereafter, 15 kg of the crude product, anhydrous ethanol, and 1 kg of purified water were added, stirred for 2 hours at room temperature, and then filtered and washed with 2 kg of anhydrous ethanol. Vacuum-drying was carried out at 50°C for 6 hours to obtain 4.25 kg of L-serine, and the chemical purity and optical purity of the product were measured to be 99.5% and 99.7%, respectively.

### Example 4-2. Production of L-serine using tartaric acid

Using (+)-tartaric acid instead of camphorsulfonic acid, 4.15 kg of L-serine was obtained by the same method as in Example 4.1. The chemical purity and optical purity of the product were measured to be 99.4% and 99.7%, respectively.

### Experimental Example 1. Confirmatory Test of L-Serine

L-serine obtained in Example 4 was analyzed based on the L-serine analysis method specified in the United States Pharmacopoeia, and it was confirmed that all of the analysis results complied with the United States Pharmacopoeia.

In a case where L-serine obtained in Example 4 was tested according to the ATR method of infrared spectrophotometry among the General Test Methods of the Korean Pharmacopoeia, it was confirmed that the main absorption is exhibited in the vicinity of each of wave numbers of 2,930 cm⁻¹, 1,676 cm⁻¹, 1,439 cm⁻¹, 1,389 cm⁻¹, 1,257 cm⁻¹, 1,093 cm⁻¹, and 658 cm⁻¹, and it was confirmed that in a case where 1 mL of a ninhydrin solution (1 -> 1,000) is added to 5 mL of a solution obtained by dissolving L-serine in water, and then heated for 3 min, the resulting solution exhibits a purple color.

In addition, as a result of analyzing the obtained L-serine with differential scanning calorimetry (DSC), it was confirmed that an endothermic peak appears at 226°C, which is consistent with the literature value (see Fig. 2).

## Claims

1. A method of producing L-serine, comprising:
a) a step of subjecting glycine and a copper compound to chelation to form a glycine-copper complex;
b) a step of subjecting a carbon source to a reaction with the complex of the a) step to form a complex into which hydroxymethyl is introduced;
c) a step of treating the complex of the b) step with a reducing agent to form DL-serine; and
d) a step of optically separating the DL-serine of the c) step to collect L-serine.

2. The method according to claim 1, wherein the copper compound in the a) step is one or more selected from the group consisting of CuSO₄, CuOAc₂, CuCl₂, CuI, CuBr, CuBr₂, and a hydrate thereof.

3. The method according to claim 1 or claim 2, wherein the a) step is carried out by adding glycine, copper, a base, and purified water.

4. The method according to claim 3, wherein the purified water is added such that the weight thereof is 6 to 30 times the weight of glycine .

5. The method according to any one of claims 1 to 4, wherein the glycine-copper complex of the a) step has the structure of Formula 2:

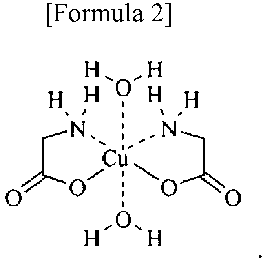

6. The method according to any one of claims 1 to 5, wherein the b) step is carried out by adding a carbon source, a base, and purified water.

7. The method according to any one of claims 1 to 6, wherein the carbon source is one or more selected from the group consisting of formaldehyde, paraformaldehyde, 1,3-dioxolane, 1,3-dioxane, and hexamidine.

8. The method according to any one of claims 1 to 7, wherein the complex of the b) step has the structure of Formula 3:

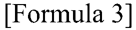

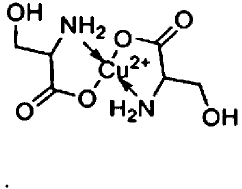

9. The method according to any one of claims 1 to 8, wherein the reducing agent in the c) step is sodium sulfide (Na₂S).

10. The method according to any one of claims 1 to 9, further comprising a step of carrying out cooling by reflux with purified water, after the c) step is carried out.

11. The method according to claim 1, wherein any one or more selected from the group consisting of tartaric acid, malic acid, mandelic acid, and camphorsulfonic acid is used in the d) step.
